# EUROPEAN PATENT APPLICATION

(11) **EP 4 159 250 A1**
(43) Date of publication of application: **05.04.2023**
(21) Application number: 22198768.8
(22) Date of filing: 29.09.2022
(51) Int. Cl.: A61L 27/24, A61L 27/38, A61L 27/52, A61L 27/60, C12N 5/00

(54) **METHOD OF ENHANCING STRUCTURAL INTEGRITY OF EPIDERMIS IN CULTURE OF RECONSTRUCTED HUMAN SKIN**

(30) Priority: 30.09.2021 KR 20210129434; 26.08.2022 KR 20220107843
(71) Applicant: Korea University Research and Business Foundation, Seoul 02841 (KR)
(72) Inventor: Chung, Seok, Seoul (KR); Won, Jihee, Seoul (KR)
(74) Representative: Schön, Christoph

(57) **Abstract**

Disclosed is a method of producing reconstructed human skin including forming a three-dimensional hydrogel scaffold matrix by gelling a matrix solution including a type I collagen solution, forming a coating layer by coating the three-dimensional hydrogel scaffold matrix with type IV collagen, and forming an epidermis by seeding epidermal keratinocytes onto the three-dimensional hydrogel scaffold matrix having the coating layer formed thereon and performing culture.

## Description

### BACKGROUND OF THE INVENTION

### Field of the Invention

The present invention relates to a method of producing reconstructed human skin, and more particularly to a method of enhancing the structural integrity of an epidermis in culture of reconstructed human skin.

### Description of the Related Art

The skin is a large organ that surrounds the body, and is broadly divided into two layers, namely the epidermis and dermis, from the outermost surface thereof. The epidermis is mostly composed of epidermal keratinocytes, which form a layered structure, and the dermis contains blood vessels, nerves, sebaceous glands, and the like based on a matrix including dermal fibroblasts inside matrix proteins such as collagen fibers and elastic fibers. In the skin, the barrier function of the epidermis is regarded as the most important, and the skin barrier function protects the body by blocking penetration of external pollutants and irritants and prevents loss of body fluids by blocking evaporation of moisture inside the body.

Reconstructed human skin (RhS) is a skin equivalent produced in a manner in which a dermis equivalent composed of a type-I-collagen-based three-dimensional hydrogel scaffold matrix including dermal fibroblasts is placed in a special culture vessel (a filter membrane insert), the bottom surface of which is formed of a porous membrane, epidermal keratinocytes are applied thereon, and the upper portion thereof is exposed to air to induce differentiation of the epidermal keratinocytes, thereby forming a mature reconstructed epidermis having a skin barrier function. This reconstructed human skin is used to replace damaged parts of the skin such as burns, external injuries, etc., or is used in skin physiology research, evaluation of skin irritation of specific materials, or evaluation of functional improvement efficacy thereof.

In the skin, the epidermis has an unique structure, stratified cell layers having morphological and functional diversity due to differentiation of epidermal keratinocytes, and consists of distinct four layers, particularly the stratum basale (SB), which proliferates to allow prolonged survival for the epidermis, the stratum spinosum (SS) and the stratum granulosum (SG), which are undergoing differentiation and transformation, and the stratum corneum (SC), which is fully differentiated and as such, only the lipid component of the cell membrane remains, forming a lipid layer and occupying a major part of the barrier function. Upon production of reconstructed human skin, forming the unique structure of the epidermis in which all the distinct four layers are present is an important criterion for determining structural integrity of the epidermis and this is evaluated by the expression of the specific markers depending on the differentiation stage of cells constituting individual layers.

However, during culture of reconstructed human skin, a dermis equivalent is formed mainly using a three-dimensional hydrogel scaffold matrix composed of type I collagen alone, without a component of a basement membrane playing an important role in structural integrity of the epidermis, which is closely related to the barrier function of the skin. In this case, there is a problem in that the stratum granulosum-limited expression of loricrin, which is considered an important marker for barrier function, is not well observed.

### SUMMARY OF THE INVENTION

The present invention has been made keeping in mind the problems encountered in the related art, and an object of the present invention is to provide a method of further enhancing structural integrity of an epidermis by adding type IV collagen, which is one of the basement membrane components, to an acellular dermal matrix or dermis equivalent serving as a substrate in order to form the epidermis in the production of reconstructed human skin.

The present invention provides a method of producing reconstructed human skin including (a) forming a three-dimensional hydrogel scaffold matrix by gelling a matrix solution including a type I collagen solution, (b) forming a coating layer by coating the three-dimensional hydrogel scaffold matrix with type IV collagen, and (c) forming an epidermis by seeding epidermal keratinocytes onto the three-dimensional hydrogel scaffold matrix having the coating layer formed thereon and performing culture.

Also, in the method according to the present invention, the matrix solution may further include dermal fibroblasts.

Also, in the method according to the present invention, the type IV collagen may be applied at a concentration of 1 to 20 µg/cm² to form the coating layer.

Also, in the method according to the present invention, step (b) may include applying a diluted type IV collagen solution in which the type IV collagen is diluted onto the three-dimensional hydrogel scaffold matrix.

Also, in the method according to the present invention, step (c) may include seeding the epidermal keratinocytes and performing culture by supplying a culture medium including the epidermal keratinocytes.

Also, in the method according to the present invention, the culture medium may be removed, and the seeded epidermal keratinocytes may be exposed to air to induce differentiation of the epidermal keratinocytes.

The features and advantages of the present invention will become more apparent from the following detailed description taken in conjunction with the accompanying drawings.

The terms or words used in the present specification and the claims should not be interpreted as being limited merely to the ordinary and dictionary meanings, but should be interpreted based on the meanings and concepts of the invention in keeping with the scope of the invention based on the principle that the inventors can appropriately define the terms in order to describe the invention in the best way.

### BRIEF DESCRIPTION OF THE DRAWINGS

FIGs. 1 and 2 are flowcharts showing a process of producing reconstructed human skin according to the present invention;
FIGs. 3A, 3B, 3C, and 3D are optical microscope images showing the histology of reconstructed human skin completed according to Comparative Example 1, Comparative Example 2, Example 1, and Example 2, respectively, stained with hematoxylin and eosin (H&E); and
FIGs. 4A, 4B, 4C, and 4D are optical microscope images showing the histology of reconstructed human skin completed according to Comparative Example 1, Comparative Example 2, Example 1, and Example 2, respectively, immunostained with a loricrin antibody.

### DETAILED DESCRIPTION OF THE INVENTION

The objects, specific advantages, and novel features of the present invention will become more apparent from the following detailed description taken in conjunction with the accompanying drawings and preferred embodiments. In the present specification, in adding reference numerals to the elements in drawings, it should be noted that the same reference numerals refer to the same elements throughout different drawings. Also, terms such as "first", "second", and the like are used to distinguish one element from another element, and the elements are not limited by these terms. Hereinafter, in describing the present invention, a detailed description of related known technology that may unnecessarily obscure the gist of the present invention will be omitted.

Hereinafter, a detailed description will be given of preferred embodiments of the present invention with reference to the accompanying drawings.

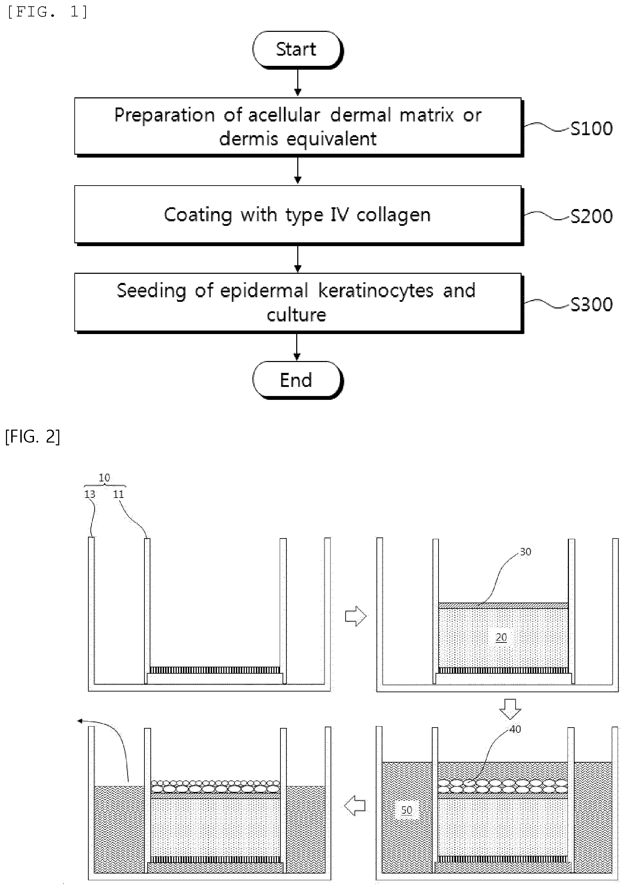

FIGs. 1 and 2 are flowcharts showing a process of producing reconstructed human skin according to the present invention.

As shown in FIGs. 1 and 2, the method of producing reconstructed human skin according to the present invention includes forming a three-dimensional hydrogel scaffold matrix 20 by gelling a matrix solution including a type I collagen solution, forming a coating layer 30 by coating the three-dimensional hydrogel scaffold matrix 20 with type IV collagen, and forming an epidermis by seeding epidermal keratinocytes 40 onto the three-dimensional hydrogel scaffold matrix 20 having the coating layer 30 formed thereon and performing culture.

The present invention pertains to a method of producing reconstructed human skin, and is intended to enhance the structural integrity of the epidermis in culture of reconstructed human skin. Here, reconstructed human skin narrowly means a skin equivalent in which an epidermis is formed on a three-dimensional hydrogel scaffold matrix based on type I collagen, which is a dermal component, and including dermal fibroblasts, and should be interpreted in the broadest sense, including all polymer composites that exhibit structural and functional properties similar to those of real skin. For example, even when the three-dimensional hydrogel scaffold matrix does not include dermal fibroblasts, the resulting product may correspond to reconstructed human skin.

Such reconstructed human skin may be cultured in a culture vessel. For example, the culture vessel 10 may be formed in a double-walled structure in which the inner chamber 11 is surrounded by the outer chamber 13. Here, the inner chamber 11 has an inner space formed by an inner wall and a bottom surface, the bottom surface may be made of a porous membrane having numerous micropores, and reconstructed human skin is cultured in the inner space, and the outer chamber 13 has an outer wall and a bottom surface, and is spaced apart from the inner chamber 11 and is configured to surround the inner chamber 11. For the sake of convenience, culturing reconstructed human skin using the culture vessel 10 is described below, but the reconstructed human skin according to the present invention does not necessarily have to be produced only using the culture vessel 10 having the above configuration.

Specifically, the method of producing reconstructed human skin according to the present invention includes forming a matrix, forming a coating layer, and forming an epidermis.

In the formation of the matrix, a three-dimensional hydrogel scaffold matrix 20 is formed. In order to form the three-dimensional hydrogel scaffold matrix 20, a matrix solution including a type I collagen solution is gelled. Here, the matrix solution may be placed in the inner chamber 11. The matrix solution may include type I collagen at a concentration of 2.0 to 6.0 mg/mL, but the concentration thereof is not necessarily limited thereto. In addition, the matrix solution may or may not include dermal fibroblasts. Thereby, the three-dimensional hydrogel scaffold matrix 20 constitutes an acellular dermal matrix not including dermal fibroblasts, or a dermis equivalent including dermal fibroblasts. In the case of including dermal fibroblasts, the number of dermal fibroblasts may be 1.0×10⁵ to 3.0×10⁵ cells/mL, but is not necessarily limited thereto.

In the formation of the coating layer, the three-dimensional hydrogel scaffold matrix 20, that is, the acellular dermal matrix or dermis equivalent, is coated with type IV collagen. Type IV collagen is a basement membrane component material of the skin, and exists as a plane between the epidermis and the dermis of the skin. The basement membrane component material is a mixture of polymer proteins found on the surface or interface of various tissues, and may be a material containing laminin, type IV collagen, heparan sulfate proteoglycans, and entactin/nidogen as main components and including various growth factors. A commercially available example thereof is Corning Matrigel^{®} isolated from mouse sarcoma. In the present invention, the coating layer 30 is formed by coating the acellular dermal matrix or dermis equivalent with type IV collagen. For example, the coating layer 30 may be formed by applying a diluted type IV collagen solution onto the type-I-collagen-based three-dimensional hydrogel scaffold matrix 20 constituting the acellular dermal matrix or dermis equivalent of reconstructed human skin. Here, type IV collagen may be applied at a concentration of 1 to 20 µg/cm². However, the concentration thereof is not necessarily limited thereto.

In the formation of the epidermis, epidermal keratinocytes 40 are seeded onto the three-dimensional hydrogel scaffold matrix 20 having the coating layer 30 formed thereon, followed by culture. Here, the number of epidermal keratinocytes 40 that are seeded may be 100,000 to 400,000 cells/cm², but may be adjusted. Specifically, a first culture medium 50 including the epidermal keratinocytes 40 is supplied to the inner chamber 11 and a first culture medium 50 not including the epidermal keratinocytes 40 is supplied to the outer chamber 13, and after incubation time (e.g. 12 to 24 hours), the epidermal keratinocytes 40 may be attached. Alternatively, after seeding the epidermal keratinocytes 40, the first culture medium 50 may be further supplied to the inner chamber 11 and the outer chamber 13 so that the epidermal keratinocytes 40 are attached.

When the epidermal keratinocytes 40 are introduced in this way, differentiation thereof may be induced to form an epidermis. In order to form an epidermis having a skin barrier function in reconstructed human skin, the culture medium may be removed from the culture vessel 10 into which the epidermal keratinocytes 40 are introduced, the epidermal keratinocytes 40 may be exposed to air, and differentiation thereof may be induced. For example, the first culture medium 50 may be removed from the culture vessel 10, and a second culture medium for differentiation of the epidermal keratinocytes 40 may be supplied to the outer chamber 13. Thereafter, differentiation may be induced for 10 days while periodically replacing the culture medium. However, the period of time for inducing differentiation is not necessarily limited to 10 days.

A better understanding of the present invention may be obtained through the following examples and experimental example.

### Example 1

As a first example of the present invention, an acellular dermal matrix including type I collagen at a concentration of 6.0 mg/mL and coated with type IV collagen was prepared. Here, the acellular dermal matrix was formed using a type-I-collagen-based three-dimensional scaffold. Specifically, in order to prepare a type I collagen solution in 1X PBS (phosphate-buffered saline) at a pH of 7.6, 6.0 mg/mL of a type I collagen solution was prepared by mixing type I collagen (Corning) with 10X PBS, 1.0 M NaOH, and distilled deionized water (DDW) in an appropriate ratio. 150 µL of the solution thus prepared was placed in a culture vessel having a porous bottom membrane, and stored for 30 minutes in an incubator at 37°C and 5% CO₂ and thus gelled. Then, 157 µL of a type IV collagen (Corning) solution at a concentration of 50 pg/mL diluted in PBS was applied onto the three-dimensional hydrogel scaffold matrix formed as described above and stored at room temperature for 1 hour, whereby the upper surface of the three-dimensional hydrogel scaffold matrix was coated with type IV collagen at a final concentration of 10 µg/cm².

### Example 2

As a second example of the present invention, a three-dimensional dermis equivalent including 6.0 mg/mL of type I collagen and 1.0×10⁵ cells/mL of dermal fibroblasts and coated with type IV collagen was prepared. Here, the dermis equivalent was formed using a type-I-collagen-based three-dimensional scaffold including dermal fibroblasts (Normal Human Dermal Fibroblasts, LONZA). Specifically, in order to prepare a type I collagen solution including dermal fibroblasts in 1X PBS (phosphate-buffered saline) at a pH of 7.6, a collagen-fibroblast-mixed solution including 6.0 mg/mL of type I collagen and 1.0×10⁵ cells/mL of dermal fibroblasts was prepared by mixing type I collagen (Corning) with 10X PBS, 1.0 M NaOH, DDW (distilled deionized water), and a cell suspension including dermal fibroblasts in an appropriate ratio. 150 µL of the solution thus obtained was placed in a culture vessel having a porous bottom membrane, and stored for 30 minutes in an incubator at 37°C and 5% CO₂ and thus gelled. 157 µL of a type IV collagen (Corning) solution at a concentration of 50 pg/mL diluted with PBS was applied onto the dermis equivalent formed as described above and stored at room temperature for 1 hour, whereby the upper surface of the dermis equivalent was coated with type IV collagen at a final concentration of 10 µg/cm².

### Comparative Example 1

As a first comparative example of the present invention, an acellular dermal matrix including 6.0 mg/mL of type I collagen was prepared. Here, a three-dimensional hydrogel scaffold matrix was formed using a type-I-collagen-based three-dimensional scaffold. Specifically, in order to prepare a type I collagen solution in 1X PBS (phosphate-buffered saline) at a pH of 7.6, 6.0 mg/mL of a type I collagen solution was prepared by mixing type I collagen (Corning) with 10X PBS, 1.0 M NaOH, DDW (distilled deionized water), and dermal fibroblasts in an appropriate ratio. 150 µL of the solution thus obtained was placed in a culture vessel having a porous bottom membrane, and stored for 30 minutes in an incubator at 37°C and 5% CO₂ and thus gelled.

### Comparative Example 2

As a second comparative example of the present invention, a three-dimensional dermis equivalent including 6.0 mg/mL of type I collagen and 1.0×10⁵ cells/mL of dermal fibroblasts was prepared. Here, the dermis equivalent was formed using a type-I-collagen-based three-dimensional scaffold including dermal fibroblasts (Normal Human Dermal Fibroblasts, LONZA). Specifically, in order to prepare a type I collagen solution in 1X PBS (phosphate-buffered saline) at a pH of 7.6, a collagen-fibroblast-mixed solution including 6.0 mg/mL of type I collagen and 1.0×10⁵ cells/mL of dermal fibroblasts was prepared by mixing type I collagen (Corning) with 10X PBS, 1.0 M NaOH, DDW (distilled deionized water), and a cell suspension including dermal fibroblasts in an appropriate ratio. 150 µL of the solution thus obtained was placed in a culture vessel having a porous bottom membrane, and stored for 30 minutes in an incubator at 37°C and 5% CO₂ and thus gelled.

### Experimental Example 1

A reconstructed epidermis was cultured on each of Examples 1 and 2 and Comparative Examples 1 and 2 through the following method.

Each of Example 1, Example 2, Comparative Example 1, and Comparative Example 2 was placed in a chamber, keratinocytes (Normal Human Epidermal Keratinocytes, LONZA) were seeded at 250,000 cells/cm² thereon, and a culture medium (Keratinocyte Growth Medium-GOLD, LONZA) was placed in the inner and outer chambers of a culture vessel, followed by cell attachment overnight. Thereafter, the culture medium was removed from the culture vessel, a culture medium for forming an epidermis was placed in the outer chamber, and differentiation of the keratinocytes was induced. Thereafter, an epidermis was produced by inducing differentiation for 10 days while replacing the culture medium every day.

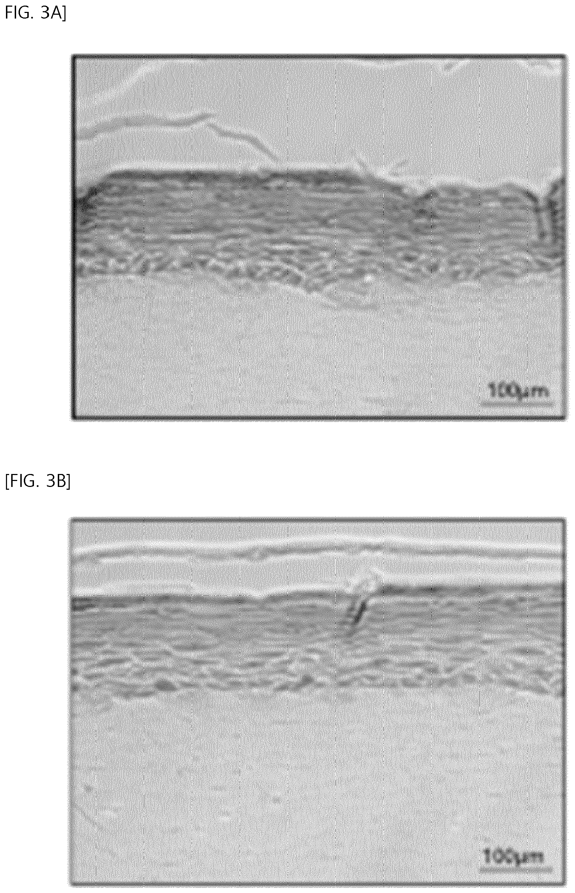

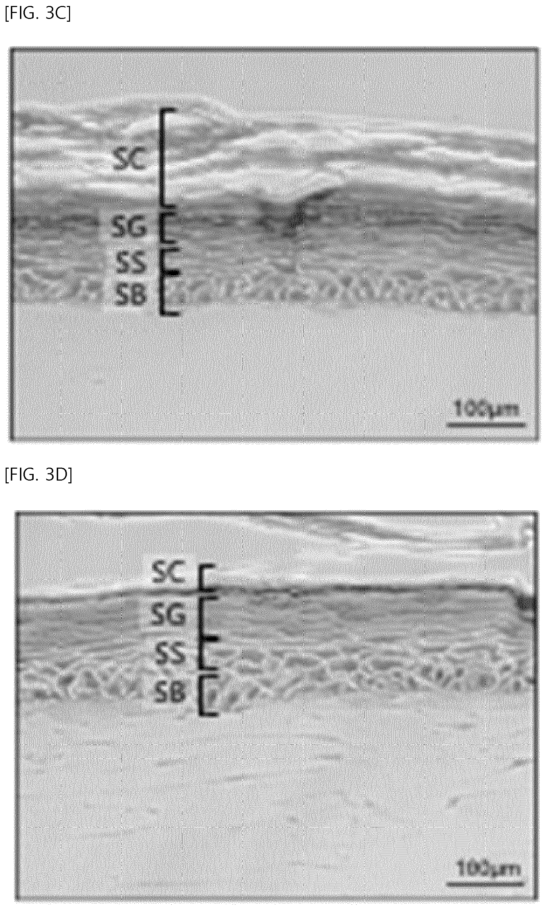

The reconstructed epidermis and reconstructed human skin were finally produced after culture as described above. Thereafter the slices of the reconstructed epidermis and reconstructed human skin were stained with hematoxylin and eosin (H&E), and observed using an optical microscope. The results thereof are shown in FIGs. 3A to 3D. FIGs. 3A, 3B, 3C, and 3D are optical microscope images showing the histology of the reconstructed human skin produced according to Comparative Example 1, Comparative Example 2, Example 1, and Example 2, respectively, stained with hematoxylin and eosin (H&E).

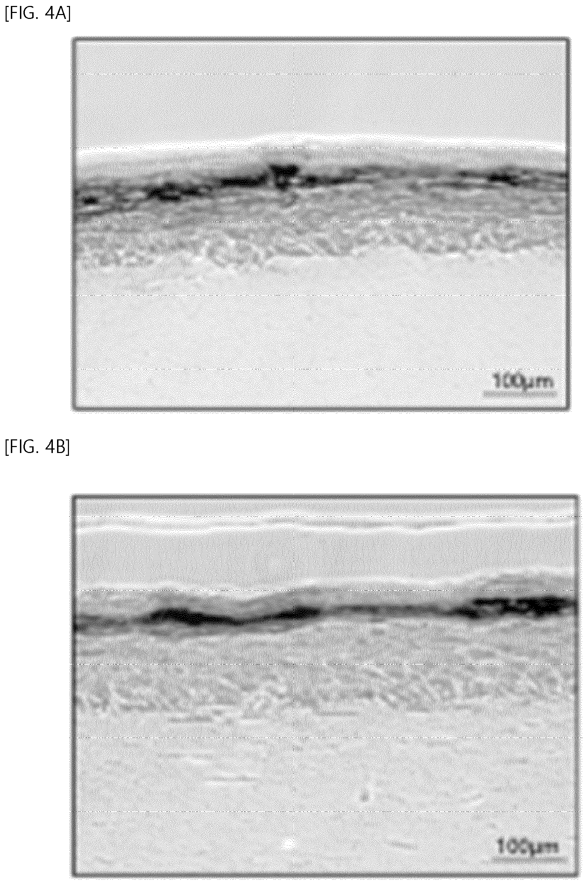

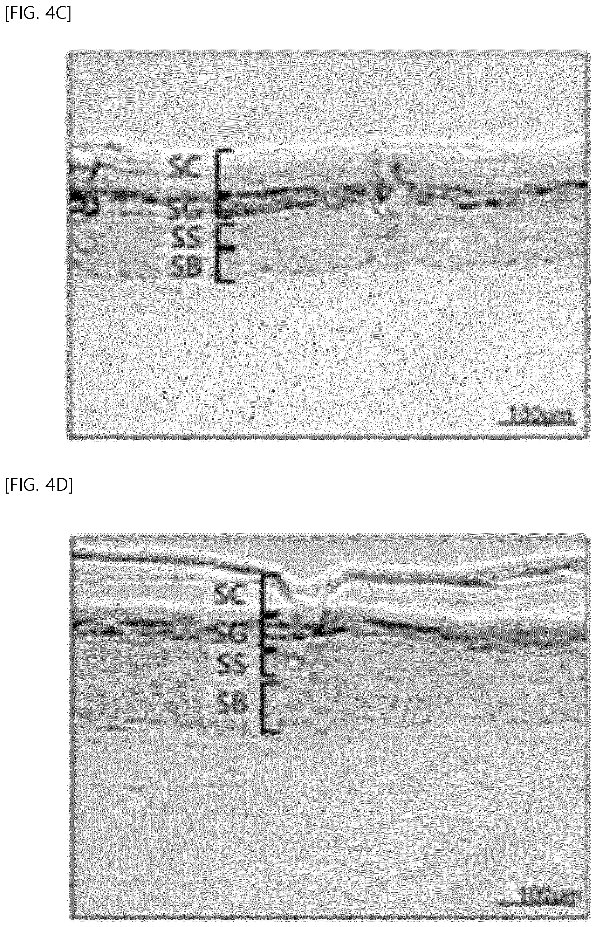

The results of immunostaining the slices of the reconstructed epidermis and reconstructed human skin finally produced after culture as described above using a loricrin antibody are shown in FIGs. 4A to 4D. FIGs. 4A, 4B, 4C, and 4D are optical microscope images showing the histology of the reconstructed human skin produced according to Comparative Example 1, Comparative Example 2, Example 1, and Example 2, respectively, immunostained with the loricrin antibody.

Consequently, as shown in FIGs. 3A to 3D, it was confirmed that the reconstructed human skin produced using Examples 1 and 2 according to the present invention had a clearly distinct layers of the epidermis compared to the reconstructed human skin produced using Comparative Examples 1 and 2.

In addition, unlike Comparative Examples 1 and 2, in which loricrin was broadly expressed in all layers as well as the stratum granulosum as shown in FIGs. 4A and 4B, Examples 1 and 2 of FIGs. 4C and 4D showed that the expression of loricrin was limited to the stratum granulosum.

This is consistent with the expression pattern of loricrin in real skin, confirming that the structural integrity of the epidermis of the reconstructed human skin produced using Examples 1 and 2 is high.

As is apparent from the above description, technology for culturing reconstructed human skin according to the present invention is capable of providing an more relevant physiology of human skin in celluar microenvironment by adding a basement membrane component to an acellular dermal matrix or dermis equivalent serving as a substrate for forming an epidermis, thereby making it possible to enhance structural integrity of the epidermis to be closer to human skin.

Since the epidermis exerts the barrier function, which is the most important functional role in the skin, structural integrity of the epidermis is determined by observing a formation of stratified structure consisting of four distinct layers, the stratum basale, the stratum spinosum, the stratum granulosum, and the stratum corneum, and furthermore, can be determined by the expression pattern of specific markers within the layers. By adding a basement membrane component to an acellular dermal matrix or dermis equivalent serving as a substrate for forming an epidermis, the expression pattern of a specific marker for each layer of the epidermis becomes similar to that of human skin, especially limited expression of a loricrin to the stratum granulosum, thereby enhancing the structural integrity of the epidermis.

Although the present invention has been described in detail through specific embodiments, these embodiments are intended to specify the present invention, and the present invention is not limited thereto, and it is obvious that modifications or improvements thereof are possible by those of ordinary skill in the art within the technical spirit of the present invention.

All simple modifications and variations of the present invention belong to the scope of the present invention, and the specific protection scope of the present invention will be made clear by the appended claims.

## Claims

1. A method of producing reconstructed human skin, comprising:
(a) forming a three-dimensional hydrogel scaffold matrix by gelling a matrix solution comprising a type I collagen solution;
(b) forming a coating layer by coating the three-dimensional hydrogel scaffold matrix with type IV collagen; and
(c) forming an epidermis by seeding epidermal keratinocytes onto the three-dimensional hydrogel scaffold matrix having the coating layer formed thereon and performing culture.

2. The method according to claim 1, wherein the matrix solution further comprises dermal fibroblasts.

3. The method according to claim 1, wherein the type IV collagen is applied at a concentration of 1 to 20 µg/cm² to form the coating layer.

4. The method according to claim 1, wherein step (b) comprises applying a diluted type IV collagen solution in which the type IV collagen is diluted onto the three-dimensional hydrogel scaffold matrix.

5. The method according to claim 1, wherein step (c) comprises seeding the epidermal keratinocytes and performing culture by supplying a culture medium comprising the epidermal keratinocytes.

6. The method according to claim 5, wherein the culture medium is removed, and the seeded epidermal keratinocytes are exposed to air so that the epidermal keratinocytes differentiate.
